# DEMANDE DE BREVET EUROPEEN

(11) **EP 1 063 007 A1**
(43) Date de publication de la demande: **27.12.2000**
(21) Numéro de dépôt: 00401246.4
(22) Date de dépôt: 05.05.2000
(51) Int. Cl.: B01F 17/00, A61K 7/48, A61K 7/00

(54) **Organogels et leurs utilisations notamment cosmétique**

(30) Priorité: 21.06.1999 FR 9907829
(71) Demandeur: L'OREAL, 75008 Paris (FR)
(72) Inventeur: Simonnet, Jean-Thierry, 75011 Paris (FR); Legret, Sylvie, 92320 Chatillon (FR)
(74) Mandataire: Rasson, Catherine

(57) **Abrégé**

La présente demande se rapporte à un organogel, c'est-à-dire à une composition sous forme d'émulsion comportant une phase huileuse dispersée dans une phase glycolique à l'aide d'un système tensioactif, caractérisée par le fait que le système tensioactif contient au moins un tensioactif non ionique susceptible de former une phase lamellaire au contact de la phase glycolique et ayant un HLB allant de 1 à 10, que la phase glycolique comprend de la glycérine et au moins un glycol et représente au moins 30 % en poids par rapport au poids total de la composition et que le rapport en poids phase glycolique/système tensioactif est égal ou supérieur à 20.

La composition obtenue a l'aspect d'un gel.

Le tensioactif apte à former une phase lamellaire au contact de la phase glycolique est de préférence choisi parmi les esters et les éthers gras de polyols.

L'invention se rapporte aussi à l'utilisation de la dite composition, en particulier pour le soin, le traitement, le nettoyage et/ou le maquillage de la peau du corps ou du visage, des cheveux et/ou des lèvres, et spécialement pour le soin des peaux sèches et/ou des lèvres sèches.

## Description

La présente invention se rapporte à un organogel, composition sous forme d'émulsion comprenant une phase huileuse dispersée dans une phase glycolique, contenant au moins un tensioactif non ionique apte à former une phase lamellaire au contact de la phase glycolique, la phase glycolique contenant de la glycérine et au moins un glycol. L'invention se rapporte aussi à l'utilisation de la dite composition, en particulier pour le soin, le traitement, le nettoyage et/ou le maquillage de la peau du corps ou du visage, des cheveux et/ou des lèvres, et spécialement pour le soin des peaux sèches et/ou des lèvres sèches.

Le terme d'organogel a été initialement utilisé pour décrire un concept particulier de gélification par une solution de gélatine, d'une microémulsion inverse eau dans huile (voir Luisi et Al. Colloid & Polymer Science, 1990, vol. 268, p. 356-374). Le terme s'est étendu depuis peu à des systèmes gélifiés comportant deux phases non miscibles (eau dans huile) stabilisées par de la lécithine enrichie en Phosphatidyl Choline (appelée ci-après PC) et le plus souvent hydrogénée (voir Williman et al. Journal of Pharmaceutical Sciences, 1992, vol. 81, p. 871-874, et Schchipunov et al., Colloid Journal, 1995, vol. 57, p. 556-560). Ces émulsions présentent une phase lamellaire et sont sous forme de gels même en l'absence de gélifiants, d'où le nom d'organogels qui désignent ce type d'émulsion quel que soit le sens de l'émulsion (E/H ou H/E).

Par la suite, il a été développé des émulsions similaires, dans lesquelles l'eau était substituée par des polyols, donnant lieu à la formation d'émulsions huile dans glycol. stabilisées par de la lécithine hydrogénée. Ce type d'émulsions comportant un taux élevé de polyols est particulièrement intéressant pour permettre une bonne hydratation de la peau à la fois par diminution de la perte insensible en eau (ou PIE) grâce au film formé par l'émulsion sur la peau et par apport d'une quantité importante de polyols, très hygroscopique, permettant l'hydratation de la peau.

Pour diverses raisons et notamment des raisons de coût, on a essayé de remplacer dans les émulsions huile-dans-polyol, la lécithine hydrogénée enrichie en PC par d'autres lécithines. Ainsi, le document JP-A-62/95132 décrit des émulsions huile dans polyols, stabilisées par des lécithines ayant subi un traitement enzymatique, et le document JP-A-62/95131 décrit une émulsion huile dans polyols. contenant une hydroxylécithine. Ces émulsions présentent l'inconvénient d'être sensibles à l'oxydation, d'être souvent coûteuses quand on utilise une lécithine traitée, et de nécessiter une forte concentration de lécithine ou l'association avec un autre tensioactif.

Par ailleurs, une autre solution a consisté à utiliser des composés autres que les dérivés de lécithine. Ainsi, le document JP-A-6/287107 décrit des émulsions de type gel, obtenues à partir d'huiles liquides, d'esters polyglycéroliques d'acides gras hydrophiles, ces esters étant nécessairement solubles dans l'eau, et de polyols, auxquels est ajouté un hyaluronate indispensable pour gélifier la composition. Dans ce cas, les tensioactifs sélectionnés du fait de leur hydrophilie ne forment pas de phase lamellaire et ne permettent pas de gélifier la dispersion.

L'introduction d'un gélifiant, dans ce cas un hyaluronate, est indispensable pour obtenir la viscosité désirée et une bonne stabilité. On n'obtient donc pas un organogel.

En outre, le document JP-A-1/301617 décrit des gels contenant de l'huile et des polyols, qui par addition d'eau, conduisent à la formation d'émulsions huile-dans-eau. Ces compositions permettent une meilleure pénétration d'actifs notamment pharmaceutiques dans la peau. Toutefois, les gels décrits dans ce document ne sont pas des émulsions huile-dans-polyol. En outre, ils contiennent souvent peu de polyols et, quand ils en contiennent une quantité importante (voir exemple 1 du document), ils contiennent aussi une forte proportion de tensioactif, ce qui peut entraîner des irritations, en particulier chez les personnes ayant une peau sensible.

Il subsiste donc le besoin d'obtenir des organogels, émulsion d'huile dans des polyols tels que glycérine et glycols, ne comportant de lécithine ou de dérivés de lécithine, en utilisant une faible quantité de tensioactif tout en ayant une forte proportion de polyols, permettant l'obtention d'émulsions gélifiées huile-dans-polyols, présentant de bonnes propriétés cosmétiques sans avoir les inconvénients de l'art antérieur.

La demanderesse a découvert de façon surprenante que les esters et éthers gras de polyols, aptes à former une phase lamellaire au contact d'une phase glycolique et ayant un HLB (Hydrophilic Lipophilic Balance) tel qu'ils sont insolubles dans l'eau, permettaient de réaliser des organogels stables et ayant des propriétés cosmétiques satisfaisantes, et ce avec un rapport en poids de la phase glycolique au système tensioactif égal ou supérieur à 20, et avec une phase glycolique contenant de la glycérine et au moins un glycol. Comme cela est bien connu, on désigne par HLB (Hydrophilic-Lipophilic Balance, au sens de Griffin ; voir J. Soc. Cosm. Chem. 1954 (vol 5), pp 249-256) l'équilibre entre le caractère hydrophile et le caractère lipophile d'un agent tensioactif.

On entend ici par « phase glycolique » le mélange de glycérine et de glycol(s). Ce mélange doit être présent en une quantité d'au moins 30% en poids par rapport au poids total de la composition, et le rapport en poids de la quantité de ce mélange sur la quantité de tensioactif(s) doit être égal ou supérieur à 20.

La présente invention se rapporte à une composition constituant un organogel sous forme d'émulsion comportant une phase huileuse dispersée dans une phase glycolique à l'aide d'un système tensioactif, caractérisée par le fait que le système tensioactif contient au moins un tensioactif non ionique ou un mélange de tensioactifs non ioniques, susceptible de former une phase lamellaire au contact de la phase glycolique et ayant un HLB allant de 1 à 10, que la phase glycolique comprend de la glycérine et au moins un glycol et représente au moins 30 % en poids par rapport au poids total de la composition et que le rapport en poids phase glycolique/système tensioactif est égal ou supérieur à 20.

La composition de l'invention présente l'avantage de ne pas nécessiter l'ajout de tensioactifs à base de lécithine et d'être bien stable malgré la faible quantité de tensioactif par rapport à la grande quantité de phase glycolique (glycérine + glycol(s)). En outre, un autre avantage provient du fait qu'il n'est pas nécessaire d'ajouter de conservateurs pour la protéger des développements bactériens, de moisissures et de champignons, du fait de la forte concentration en glycols et de la faible activité en eau (aw).

Les tensioactifs non ioniques susceptibles de former une phase lamellaire au contact de la phase glycolique, utilisés dans la composition de l'invention, sont amphiphiles et doivent ne pas être solubles dans l'eau. Ils ont un HLB (Hydrophilic Lipophilic Balance) allant de 1 à 10 et de préférence de 2 à 8. On peut utiliser un tensioactif seul ou un mélange de tensioactifs. Quand on utilise un mélange de tensioactifs, les différents tensioactifs constituant le mélange peuvent avoir un HLB en dehors de la fourchette 1-10 du moment que le mélange de tensioactifs non ioniques a un HLB allant de 1 à 10.

Ces tensioactifs sont préférentiellement choisis parmi les esters gras de polyols, les éthers gras de polyols, les mélanges d'esters gras de polyols et les mélanges d'éthers gras de polyols. Les esters sont formés d'au moins un polyol et d'au moins un acide gras. L'acide gras peut comporter de 8 à 22 atomes de carbone et peut avoir une chaîne saturée ou non saturée, linéaire ou ramifiée. De préférence, l'acide gras a une chaîne saturée. Cet acide gras est choisi de préférence parmi l'acide palmitique, l'acide stéarique et leurs mélanges. Les tensioactifs utilisés dans la composition de l'invention peuvent comporter de 1 à 10 chaînes et de préférence de 1 à 3 chaînes d'acides gras.

Les éthers gras de polyols sont formés d'au moins un polyol et d'au moins un alcool gras. L'alcool gras peut comporter de 8 à 22 atomes de carbone et peut avoir une chaîne saturée ou non saturée, linéaire ou ramifiée. De préférence, l'alcool gras a une chaîne saturée. Cet alcool gras est choisi de préférence parmi l'alcool cétylique, l'alcool stéarylique, l'alcool laurylique et leurs mélanges.

Le polyol constitutif des tensioactifs utilisés dans la composition de l'invention est de préférence choisi dans le groupe formé par le glycérol, les polyglycérols, le sorbitan, les polysorbitans, les dérivés de sorbitan polyoxyéthylénés et notamment ceux comportant de 1 à 20 unités d'oxyde d'éthylène, les polyéthylènes (PEG) et notamment ceux comportant de 1 à 40 unités d'oxyde d'éthylène, le sucrose, le glucose, les dérivés oxyéthylénés du glucose et notamment ceux comportant de 1 à 20 unités d'oxyde d'éthylène, les polyglucoses. les polyglucoses oxyéthylénés et notamment ceux comportant de 1 à 20 unités d'oxyde d'éthylène, et leurs mélanges.

Les tensioactifs utilisés dans le cadre de la présente invention peuvent être notamment choisis parmi les dérivés polyoxyéthylénés des alcools cétylique, stéarylique et/ou laurylique, les esters de sorbitan et d'acide stéarique, les esters de sorbitan et d'acide palmitique, les esters de sucrose et d'acide stéarique, les esters de polyéthylène glycol de l'acide stéarique, les esters de glycérine ou de polyglycérine et de l'acide stéarique et leurs mélanges.

A titre d'exemple, on peut citer comme tensioactifs susceptibles de former une phase lamellaire avec la phase glycolique, les composés suivants (en nom CTFA, International Cosmetic Ingrédient Dictionary and Handbook) : Laureth-7 (par exemple BRIJ 30 commercialisé par la société ICI). Ceteth-2 (par exemple BRIJ 52 commercialisé par la société ICI), Steareth-2 (par exemple BRIJ 72 commercialisé par la société ICI), Sorbitan palmitate (par exemple SPAN 40 commercialisé par la société ICI), Sorbitan stearate (par exemple SPAN 60 commercialisé par la société ICI), Sorbitan tristearate (par exemple SPAN 65 commercialisé par la société ICI), PEG-8 stearate (par exemple MYRJ 45 commercialisé par la société chez ICI), Sucrose distearate qui est un mélange de mono, di et tri ester de sucrose (par exemple CRODESTA F10, CRODESTA F20, CRODESTA F50 commercialisés par la société Croda). Polyglyceryl-2 stearate (par exemple NIKKOL DGMS commercialisé par la société Nikko), Polyglyceryl-2 distearate (par exemple EMALEX PSGA commercialisé par la société Nikko), Polyglyceryl-3 distearate (par exemple EMALEX DGS 3 commercialisé par la société Nikko).

Le système tensioactif de la composition selon l'invention comprend, comme indiqué ci-dessus, au moins un tensioactif non ionique ou un mélange de tensioactifs non ioniques. Il peut aussi comprendre un mélange de tensioactif(s) non ionique(s) et ionique(s), l'addition de tensioactif ionique permettant si nécessaire, d'améliorer la stabilité de l'émulsion. Toutefois, le tensioactif ionique ne doit pas modifier la capacité du tensioactif non ionique à former une phase lamellaire.

Dans la composition de l'invention, la quantité du système tensioactif va de préférence de 0,1 à 4,25 % et mieux de 0.5 à 3 % en poids par rapport au poids total de la composition.

Le rapport en poids phase glycolique/système tensioactif est égal ou supérieur à 20 et va de préférence de 20 à 50. Comme indiqué ci-dessus, on entend ici par « phase glycolique » le mélange de glycérine et de glycols. C'est le rapport en poids de la quantité de ce mélange sur la quantité de tensioactif qui doit être égal ou supérieur à 20.

Les tensioactifs ioniques utilisables dans la composition de l'invention peuvent être choisis parmi les tensioactifs anioniques de préférence neutralisés, les dérivés alkylsulfoniques, les tensioactifs cationiques et leurs mélanges.

Les tensioactifs anioniques sont plus particulièrement choisis dans le groupe formé par :
- le dicetylphosphate, le dimyristylphosphate et leurs sels alcalins ;
- les sels alcalins du cholestérol sulfate ;
- les sels alcalins du cholestérol phosphate ;
- les lipoaminoacides tels que les acylglutamates mono- et di-sodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les phospholipides.

Les dérivés alkylsulfoniques peuvent être plus particulièrement choisis parmi les dérivés alkylsulfoniques de formule (I) : 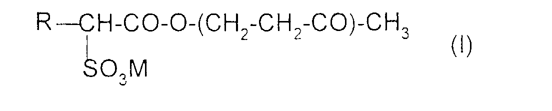 dans laquelle R représente un radical alkyle comportant de 16 à 22 atomes de carbone, en particulier les radicaux C₁₆H₃₃ et C₁₈H₃₇ pris en mélange ou séparément, et M est un métal alcalin tel que le sodium.

Les tensioactifs cationiques peuvent être choisis, par exemple dans le groupe formé par les sels d'ammonium quaternaire, les amines grasses et leurs sels.

Les sels d'ammonium quaternaires sont par exemple :
- ceux qui présentent la formule générale (Il) suivante : 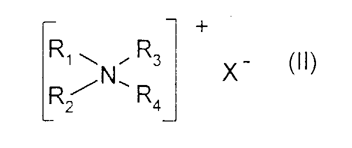 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₂-C₆)sulfates, alkyl-ou-alkylarylsulfonates. Comme sels d'ammonium quaternaire de formule (Il), on préfère, d'une part, les chlorures de tétraalkylammonium comme par exemple les chlorures de dialkyldiméthylammonium ou d'alkyltriméthylammonium, dans lesquels le radical alkyle comporte environ de 12 à 22 atomes de carbone, en particulier les chlorures de béhényltriméthylammonium, de distéaryldiméthylammonium, de cétyltriméthyl-ammonium, de benzyl diméthyl stéaryl-ammonium ou encore, d'autre part, le chlorure de stéaramidopropyldiméthyl (myristyl acetate) ammonium vendu sous la dénomination CERAPHYL 70 par la société Van Dyk.
- les sels d'ammonium quaternaire de l'imidazolinium, comme par exemple ceux de formule (III) suivante : 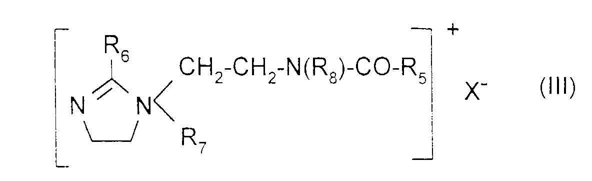 dans laquelle R₅ représente un radical alcényle ou alkyle comportant de 8 à 30 atomes de carbone, par exemple dérivé des acides gras du suif ; R₆ représente un atome d'hydrogène, un radical alkyle comportant de 1 à 4 atomes de carbone ou un radical alcényle ou alkyle comportant de 8 à 30 atomes de carbone ; R₇ représente un radical alkyle comportant de 1 à 4 atomes de carbone ; R₈ représente un atome d'hydrogène, un radical alkyle comportant de 1 à 4 atomes de carbone ; X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone, par exemple dérivés des acides gras du suif, R₇ désigne un radical méthyle, R₈ désigne l'hydrogène. Un tel produit est par exemple vendu sous la dénomination REWOQUAT W 75 par la société Rewo.
- les sels de diammonium quaternaire de formule (IV) : 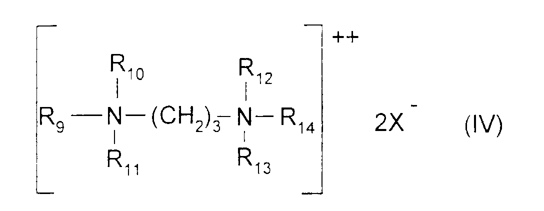 dans laquelle R₆ désigne un radical aliphatique comportant environ de 16 à 30 atomes de carbone ; R₇, R₈, R₉, R₁₀, et R₁₁ sont choisis parmi l'hydrogène ou un radical alkyle comportant de 1 à 4 atomes de carbone ; et X est un anion choisi dans le groupe des halogénures, acétates, phosphates, nitrates et méthylsulfates. De tels sels de diammonium quaternaire comprennent notamment le dichlorure de propanesuif diammonium.

Le ou les tensioactifs ioniques quand ils sont présents peuvent l'être en une quantité d'au maximum 20 % en poids par rapport au poids de tensioactif(s) non ionique(s) utilisé(s). Quand la composition en contient, la quantité de tensioactif(s) ionique(s) va de préférence de 0,01 à 5 % en poids par rapport au poids total du système tensioactif.

La phase glycolique contient au moins de la glycérine et au moins un glycol. Une telle association est nécessaire pour avoir une bonne stabilité de l'émulsion. En effet, la glycérine utilisée seule ne permet pas d'obtenir un organogel stable à long terme et il faut lui adjoindre au moins un glycol pour avoir une stabilité satisfaisante.

Les glycols utilisés dans la composition de l'invention peuvent être choisis par exemple parmi le propylène glycol, le 1,3 butylène glycol, le dipropylène glycol, le pentylène glycol, l'isoprène glycol et les polyéthylène glycols notamment ceux comportant 4 à 16 unités d'oxyde d'éthylène et de préférence de 8 à 12 unités d'oxyde d'éthylène, et leurs mélanges.

La phase glycolique (mélange glycérine + glycols) représente, dans la composition de l'invention, au moins 30 % en poids par rapport au poids total de la composition, et de préférence de 30 à 85 % et mieux de 40 à 75 % en poids par rapport au poids total de la composition. Selon un mode préféré de réalisation de l'invention, la glycérine et le ou les glycols sont présents en un rapport en poids glycérine/glycols allant de 0,5 à 5 et de préférence de 1 à 5. La glycérine représente de préférence de 20 à 60 % en poids du poids total de la composition, et le ou les glycols représentent de préférence de 10 à 40 % en poids du poids total de la composition.

Il est possible d'introduire de l'eau dans l'émulsion de l'invention, la phase glycolique devient alors une phase hydroglycolique. De préférence, la quantité d'eau est telle que l'activité en eau (aw) de la composition finale n'est pas supérieure à 0,7. On peut utiliser différentes méthodes pour mesurer l'activité en eau d'une composition, la plus courante étant la méthode manométrique par laquelle on mesure directement la pression de vapeur. De préférence, la quantité d'eau dans la composition de l'invention est au maximum de 40 % en poids par rapport au poids total de la composition.

La phase huileuse de la composition selon l'invention représente généralement de 5 à 45 % et de préférence de 15 à 35 % en poids par rapport au poids total de la composition.

La nature de la phase huileuse de l'émulsion selon l'invention n'est pas critique. La phase huileuse peut ainsi être constituée par tous les corps gras et notamment les huiles, classiquement utilisés dans les domaines cosmétique ou dermatologique.

Parmi les huiles utilisables dans l'émulsion de l'invention, on peut citer par exemple les huiles d'origine végétale, telles que les huiles de jojoba, avocat, amande douce, abricot, maïs et la fraction liquide de beurre de karité ; les huiles minérales comme l'huile de vaseline ; les huiles de synthèse comme les triglycérides (par exemple caprylic/capric triglycerides), le palmitate d'éthyl-2 hexyle, le myristate d'isopropyle, l'isoparaffine hydrogénée, l'isononanoate d'isononyle, l'octanoate de cétéaryle ; les huiles de silicone volatiles ou non volatiles et les huiles fluorées. Les autres corps gras susceptibles d'être présents dans la phase huileuse peuvent être par exemple les acides gras, les alcools gras, les cires et les lipides tels que les céramides.

La composition selon l'invention peut constituer notamment une composition cosmétique ou dermatologique et contient alors un milieu physiologiquement acceptable, c'est-à-dire compatible avec la peau, les lèvres, le cuir chevelu, les yeux et/ou les cheveux.

De façon connue, les compositions de l'invention peuvent contenir des adjuvants habituels dans les domaines considérés, tels que des actifs (par exemple acétate de tocophérol), des gélifiants, des conservateurs, des antioxydants, des parfums, des solvants, des charges, des filtres, des matières colorantes, des agents basiques ou acides et encore des vésicules lipidiques. Ces adjuvants sont utilisés dans les proportions habituelles dans les domaines considérés, et par exemple de 0.01 à 30 % du poids total de la composition, et ils sont, selon leur nature. introduits dans l'une ou l'autre phase de l'émulsion, ou encore dans des vésicules. Ces adjuvants ainsi que leurs concentrations doivent être tels qu'ils ne modifient pas la propriété recherchée pour l'émulsion de l'invention.

L'addition de charges permet de modifier, si besoin est, la texture de la composition. Comme charges qui peuvent être utilisées dans la composition de l'invention, on peut citer par exemple, outre les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les poudres de matériaux organiques naturels tels que les amidons de maïs, de blé ou de riz. réticulés ou non, telles que les poudres d'amidon réticulé par l'anhydride octénylsuccinate, commercialisées sous la dénomination DRY-FLO par la société National Starch : les microbilles de résine de silicone telles que celles commercialisées sous la dénomination TOSPEARL par la société Toshiba Silicone ; et leurs mélanges. Ces charges peuvent être présentes dans une quantité allant de 0 à 10 % en poids et de préférence de 0,5 à 4 % en poids par rapport au poids total de la composition.

Le tensioactif non ionique utilisé dans la composition de l'invention permet d'obtenir une émulsion stable. Toutefois, selon la quantité et la nature du tensioactif, on peut être amené à corriger les variations de viscosité par l'introduction de gélifiants. Ainsi, il est possible d'introduire dans la composition de l'invention, un gélifiant hydrophile ou lipophile, à la condition qu'il soit en mesure de gonfler dans la phase dans laquelle il est dispersé. Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles, l'ADN et les argiles, et comme gélifiants lipophiles, on peut citer les argiles modifiées comme les bentones, les sels métalliques d'acides gras, la silice hydrophobe et les polyéthylènes.

Les compositions selon l'invention sont opalescentes à opaques et peuvent être plus ou moins fluides. Elles peuvent donc se présenter sous forme de sérum, de lait, de crème ou de pâte. Ces compositions sont préparées selon les méthodes usuelles.

Les compositions, objets de l'invention, trouvent notamment leur application dans un grand nombre de traitements cosmétiques de la peau, des lèvres et des cheveux, y compris le cuir chevelu, notamment pour le traitement, la protection, le soin, le démaquillage et/ou le nettoyage de la peau, des lèvres et/ou des cheveux, et/ou pour le maquillage de la peau et/ou des lèvres. Du fait de leur propriété hydratante, elles peuvent être destinées aussi au traitement des peaux sèches et/ou des lèvres sèches.

Les compositions selon l'invention peuvent par exemple être utilisées comme produits de soin, de démaquillage et/ou de nettoyage pour le visage sous forme de crèmes ou de laits ou comme produits de maquillage (peau et lèvres) par incorporation de charges ou de colorants. Il est également possible de les utiliser comme masque de nettoyage, qui s'émulsifie avec l'eau de rinçage permettant d'avoir une action hydratante et de soin pour la peau.

En outre, du fait qu'elles peuvent être exemptes ou pratiquement exemptes de conservateurs, les compositions de l'invention conviennent particulièrement pour les peaux sensibles.

Aussi, l'invention a encore pour objet l'utilisation cosmétique de la composition telle que définie ci-dessus pour le traitement, la protection, le soin, le démaquillage et/ou le nettoyage de la peau. des lèvres et/ou des cheveux, et/ou pour le maquillage de la peau et/ou des lèvres.

L'invention a aussi pour objet l'utilisation cosmétique de la composition telle que définie ci-dessus, comme masque.

L'invention a aussi pour objet un procédé de traitement cosmétique de la peau, y compris du cuir chevelu, des cheveux, et/ou des lèvres, caractérisé par le fait qu'on applique sur la peau, les cheveux et/ou les lèvres, une composition telle que définie ci-dessus.

L'invention a aussi pour objet l'utilisation de la composition telle que définie ci-dessus pour la fabrication d'une composition destinée au soin des peaux sèches et/ou des lèvres sèches et/ou des peaux sensibles.

Les exemples qui suivent permettront de mieux comprendre l'invention, sans toutefois présenter un caractère limitatif. Les quantités indiquées sont en % en poids, sauf mention contraire.

### Exemple 1 : Organogel

| | |
|---|---|
| Sorbitan stearate (SPAN 60 de la société ICI) | 2 % |
| Glycérol | 50 % |
| Dipropylène glycol | 20 % |
| Huile d'abricot | 27 % |
| Acétate de tocophérol | 1 % |

Mode opératoire : On introduit sous vive agitation à 75°C, le Span 60 dans le mélange de glycérol et de dipropylène glycol. L'agitation est maintenue dans les mêmes conditions pendant 30 minutes. La phase huileuse est ensuite ajoutée, toujours dans les mêmes conditions qui sont maintenues pendant encore une heure. Puis. on arrête le chauffage et on réduit la vitesse d'agitation jusqu'à ce que la température soit descendue à 60°C. On passe ensuite à l'ultrarax à environ 20 000 tours/minute pendant 2 minutes à une température supérieure d'environ 5°C à la température de transition de phase du tensioactif, comprise pour le Span 60 entre 45 et 55°C. Le réseau lamellaire se forme dans les 24 heures.

On obtient une émulsion gélifiée fluide, très fine et stable, ayant une aw de 0,066.

L'émulsion obtenue peut être utilisée par exemple pour le soin et l'hydratation de la peau et du contour des yeux.

### Exemple 2 : Organogel

| | |
|---|---|
| Polyglycerol distearate (EMALEX DGS 3 de la société Nikko) | 2 % |
| Glycérol | 36 % |
| Dipropylène glycol | 14 % |
| Caprylic/capric triglyceride | 27 % |
| Acétate de tocophérol | 1 % |
| Eau distillée | 20 % |

Mode opératoire : On introduit sous vive agitation à 75°C, l'Emalex DGS 3 dans le mélange de glycérol et de dipropylène glycol. L'agitation est maintenue dans les mêmes conditions pendant 30 minutes. La phase huileuse est ensuite ajoutée, toujours dans les mêmes conditions qui sont maintenues pendant encore une heure. Puis, on arrête le chauffage et on réduit la vitesse d'agitation jusqu'à ce que la température soit descendue à 60°C. On passe ensuite à l'ultrarax à environ 20 000 tours/minute pendant 2 minutes à une température supérieure d'environ 5°C à la température de transition de phase du tensioactif, comprise pour l'EMALEX DGS 3 entre 45 et 55°C. Puis on ajoute l'eau. Le réseau lamellaire se forme dans les 24 heures.

On obtient une émulsion gélifiée fluide, très fine et stable, ayant une aw de 0,542.

L'émulsion obtenue peut être utilisée comme fluide de soin pour peau très sèche.

## Revendications

1. Composition constituant un organogel sous forme d'émulsion comportant une phase huileuse dispersée dans une phase glycolique à l'aide d'un système tensioactif, caractérisée en ce que le système tensioactif contient au moins un tensioactif non ionique ou un mélange de tensioactifs non ioniques, susceptible de former une phase lamellaire au contact de la phase glycolique et ayant un HLB allant de 1 à 10, que la phase glycolique comprend de la glycérine et au moins un glycol et représente au moins 30 % en poids par rapport au poids total de la composition et que le rapport en poids phase glycolique/système tensioactif est égal ou supérieur à 20.

2. Composition selon la revendication 1, caractérisée en ce que le tensioactif non ionique est choisi parmi les esters gras de polyols, les éthers gras de polyols, les mélanges d'esters gras de polyols et les mélanges d'éthers gras de polyols.

3. Composition selon la revendication précédente, caractérisée en ce que l'ester gras de polyol est formé d'au moins un polyol et d'au moins un acide gras comportant de 8 à 22 atomes de carbone et ayant une chaîne saturée ou non saturée, linéaire ou ramifiée.

4. Composition selon la revendication précédente, caractérisée en ce que l'acide gras est choisi parmi l'acide palmitique, de l'acide stéarique et leurs mélanges.

5. Composition selon la revendication 2, caractérisée en ce que l'éther gras de polyol est formé d'au moins un polyol et d'au moins un alcool gras comportant de 8 à 22 atomes de carbone et ayant une chaîne saturée ou non saturée, linéaire ou ramifiée.

6. Composition selon la revendication précédente, caractérisée en ce que l'alcool gras est choisi parmi l'alcool cétylique, l'alcool stéarylique, l'alcool laurylique et leurs mélanges.

7. Composition selon l'une quelconque des revendications 2 à 6, caractérisée en ce que l'ester gras de polyol ou l'éther gras de polyol sont formés à partir d'un polyol choisi dans le groupe comprenant le glycérol, les polyglycérols, le sorbitan, les polysorbitans, les dérivés de sorbitan polyoxyéthylénés, les polyéthylènes, le sucrose, le glucose, les dérivés oxyéthylénés du glucose, les polyglucoses, les polyglucoses oxyéthylénés et leurs mélanges.

8. Composition selon l'une quelconque des revendications précédentes, caractérisée en ce que le tensioactif non ionique est choisi parmi les dérivés polyoxyéthylénés des alcools cétylique, stéarylique et/ou laurylique, les esters de sorbitan et d'acide stéarique, les esters de sorbitan et d'acide palmitique, les esters de sucrose et d'acide stéarique, les esters de polyéthylène glycol de l'acide stéarique, les esters de glycérine ou de polyglycérine et d'acide stéarique, et leurs mélanges.

9. Composition selon l'une quelconque des revendications précédentes, caractérisée en ce que le système tensioactif contient en outre au moins un tensioactif ionique.

10. Composition selon la revendication précédente, caractérisée en ce que la quantité de tensioactif(s) ionique(s) est d'au maximum 20 % en poids par rapport au poids de tensioactif non ionique susceptible de former une phase lamellaire.

11. Composition selon l'une quelconque des revendications précédentes, caractérisée en ce que la quantité du système tensioactif va de 0,1 à 4,25 % en poids par rapport au poids total de la composition.

12. Composition selon l'une quelconque des revendications précédentes, caractérisée en ce que les glycols sont choisis parmi le propylène glycol, le 1,3 butylène glycol, le dipropylène glycol, le pentylène glycol, l'isoprène glycol et les polyéthylène glycols et leurs mélanges.

13. Composition selon l'une quelconque des revendications précédentes, caractérisée en ce que la phase glycolique représente de 30 à 85 % en poids par rapport au poids total de la composition.

14. Composition selon l'une quelconque des revendications précédentes, caractérisée en ce que le rapport en poids glycérine/glycols va de 0,5 à 5.

15. Composition selon l'une quelconque des revendications précédentes, caractérisée en ce qu'elle contient de l'eau.

16. Composition selon l'une quelconque des revendications précédentes, caractérisée en ce que la phase huileuse représente de 5 à 45 % en poids par rapport au poids total de la composition.

17. Composition selon l'une quelconque des revendications précédentes, caractérisée en ce qu'elle constitue une composition cosmétique et/ou dermatologique.

18. Composition selon l'une quelconque des revendications précédentes, caractérisée en ce qu'elle contient un adjuvant choisi parmi les actifs, les gélifiants, les conservateurs, les antioxydants, les parfums, les solvants, les charges, les filtres, les matières colorantes, les agents basiques ou acides, les vésicules lipidiques.

19. Utilisation cosmétique de la composition selon l'une quelconque des revendications 1 à 18, pour le traitement, la protection, le soin, le démaquillage et/ou le nettoyage de la peau. des lèvres et/ou des cheveux, et/ou pour le maquillage de la peau et/ou des lèvres.

20. Procédé de traitement cosmétique de la peau, des cheveux, et/ou des lèvres, caractérisé en ce qu'on applique sur la peau, les cheveux et/ou les lèvres, une composition selon l'une quelconque des revendications 1 à 18.

21. Utilisation de la composition selon l'une quelconque des revendications 1 à 18, pour la fabrication d'une composition destinée au soin des peaux sèches et/ou des lèvres sèches et/ou des peaux sensibles.

22. Utilisation cosmétique de la composition selon l'une quelconque des revendications 1 à 18, comme masque.
